(19) Europäisches Patentamt
European Patent Office
Office européen des brevets

(11) Veröffentlichungsnummer: **0 338 352 A2**

(12) **EUROPÄISCHE PATENTANMELDUNG**

(21) Anmeldenummer: 89106225.9

(22) Anmeldetag: 08.04.89

(51) Int. Cl.4: C07D 277/42 , A61K 31/625 , C07D 307/24 , C07D 211/58 , C07D 217/04 , C07D 233/60 , C07D 233/68 , C07D 209/14

(30) Priorität: 16.04.88 DE 3812755

(43) Veröffentlichungstag der Anmeldung: 25.10.89 Patentblatt 89/43

(84) Benannte Vertragsstaaten: AT BE CH DE ES FR GB IT LI NL SE

(71) Anmelder: **MERCK PATENT GESELLSCHAFT MIT BESCHRÄNKTER HAFTUNG**
Frankfurter Strasse 250 Postfach 4119
D-6100 Darmstadt(DE)

(72) Erfinder: Kurmeier, Hans-Adolf, Dr.
Hinter der Schule 3a
D-6104 Seeheim-Jugenheim(DE)
Erfinder: Weber, Wolf-Dietrich, Dr.
Lindenweg 32
D-6107 Reinheim(DE)
Erfinder: Radunz, Hans-Eckart, Dr.
Am Klingenteich 5
D-6109 Mühltal(DE)
Erfinder: Schliep, Hans-Jochen, Dr.
Weingartenstrasse 16
D-6109 Traisa(DE)

(54) **Salicylsäurederivate.**

(57) Neue Salicylsäurederivate der allgemeinen Formel I

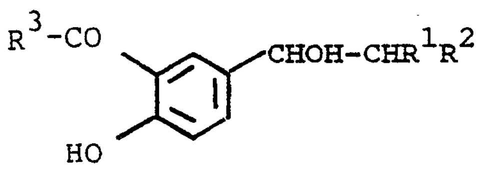

I

worin
$R^1$ H oder $CH_3$,
$R^2$ 4-(4-Methyl-2-thiazolyl)-piperazino, 4-(Tetrahydro-2-furoyl)-piperazino, 4-(4-Methyl-2-thiazolyl)-homopiperazino, 4-Benzamidopiperidino, 6,7-Dimethoxy-1,2,3,4-tetrahydroisochinolino, 1-Imidazolyl, Tribrom-1-imidazolyl oder 2-(3-Indolyl)-1,1-dimethyl-ethylamino und
$R^3$ Alkoxy mit 1 - 4 C-Atomen, $NH_2$ oder Alkylamino mit 1 - 4 C-Atomen
bedeuten,
sowie deren physiologisch unbedenkliche Säureadditionssalze zeigen Wirkungen auf den Kreislauf, insbesondere blutdrucksenkende, herzentlastende und diuretische Wirkungen.

EP 0 338 352 A2

## Salicylsäurederivate

Die Erfindung betrifft neue Salicylsäurederivate der allgemeinen Formel I

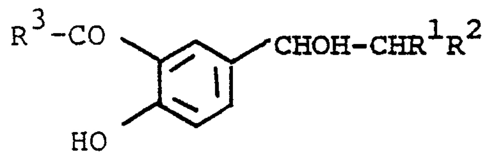

I

worin

$R^1$ H oder $CH_3$,

$R^2$ 4-(4-Methyl-2-thiazolyl)-piperazino,
4-(Tetrahydro-2-furoyl)-piperazino,
4-(4-Methyl-2-thiazolyl)-homopiperazino,
4-Benzamidopiperidino,
6,7-Dimethoxy-1,2,3,4-tetrahydroisochinolino,
1-Imidazolyl, Tribrom-1-imidazolyl oder 2-(3-Indolyl)-1,1-dimethyl-ethylamino und

$R^3$ Alkoxy mit 1 - 4 C-Atomen, $NH_2$ oder Alkylamino mit 1 - 4 C-Atomen

bedeuten,
sowie deren physiologisch unbedenkliche Säureadditionssalze.

Ähnliche Verbindungen sind in der DE-OS 2144 080 und in der DE-OS 23 02 717 beschrieben.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze bei guter Verträglichkeit wertvolle pharmakologische Eigenschaften besitzen. So zeigen sich beispielsweise Wirkungen auf den Kreislauf, insbesondere blutdrucksenkende und herzentlastende, somit kardioprotektive Wirkungen.

Die Substanzen vermindern z. B. den an der Carotisschlinge des wachen mischrassigen Hundes gemessenen Blutdruck (Methodik vgl. E.C. van LEERSUM, Pflügers Archiv 142, 377-395 (1911) bei nephrogen hypertonen Tieren (Methodik vgl. I.H. PAGE, Science 89, 273-274 (1939) im 10-Tage-Dauerversuch bei oraler Gabe von Dosen, die niedriger als 2 mg/kg/Tag liegen können, dosisabhängig auf ein erniedrigtes Niveau.

Weiterhin wird der bei katheter-tragenden (Methode vgl. J.R. WEEKS und J.A. JONES, Proc.Soc.Exptl.Biol.Med. 104, 646-648 (1960)) wachen spontan hypertonen Ratten (Stamm SHR/NIH-MO/CHB-EMD) direkt gemessene arterielle Blutdruck nach einmaliger intragastraler Gabe ab 10 mg/kg dosisabhängig gesenkt.

Die herzentlastende Wirkung läßt sich aus den folgenden am narkotisierten Hund nach intravenöser Gabe beobachteten kardiovaskulären Wirkungen ableiten: (1) Zunahme der gesamt vaskulären Kapazität (Methode: H. SUGA, et al. Pflügers Archiy, 361, 95-98 (1975). (2) Nachlastsenkung und Abnahme der Herzleistung (Methode: K. STEPHAN, et al. Arzneimittelforschung, 25, 1770-1776 (1975)). Die durch die erfindungsgemäßen Substanzen gesteigerte Ischämie-Toleranz des Herzens (Kardioprotektion) läßt sich am narkotisierten Hund mit der Methode der reversiblen Coronararterien-Okklusion (P.R. MAROKO et al. Circulation, 43, 67-82 (1971)) und am narkotisierten Schwein mit eingeschränkter Coronardurchblutung (Methode: P.D. VERDOUW et al. European Heart Journal, 4, (suppl. C), 61-67 (1983)) nachweisen.

Die Verbindungen der Formel I und ihre physiologisch unbedenklichen Säureadditionssalze können daher als Arzneimittelwirkstoffe verwendet werden. Insbesondere dienen die Ester der Formel I ($R^3$ = Alkoxy mit 1-4 C-Atomen) auch als Zwischenprodukte zur Herstellung der Amide der Formel I.

Gegenstand der Erfindung sind die Verbindungen der Formel I und deren physiologisch unbedenkliche Säureadditionssalze.

In der Formel I bedeutet der Rest $R^1$ bevorzugt H. Der Rest $R^2$ ist bevorzugt 4-(4-Methyl-2-thiazolyl)-piperazino. $R^3$ steht vorzugsweise für eine $NH_2$-Gruppe.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Eine bevorzugte Gruppe von Verbindungen entspricht der Formel I, worin $R^1$ und $R^3$ die

angegebenen Bedeutungen haben und $R^2$ 4-(4-Methyl-2-thiazolyl)-piperazino, 4-(Tetrahydro-2-furoyl)-piperazino, 4-(4-Methyl-2-thiazolyl)-homopiperazino, 6,7-Dimethoxy-1,2,3,4-tetrahydroisochinolino, 1-Imidazolyl, Tribrom-1-imidazolyl oder 2-(3-Indolyl)-1,1-dimethyl-ethylamino bedeutet.

Gegenstand der Erfindung ist ferner ein Verfahren zur Herstellung der Verbindungen der Formel I sowie ihrer physiologisch unbedenklichen Säureadditionssalze, dadurch gekennzeichnet, daß man ein Salicylsäurederivat der allgemeinen Formel II

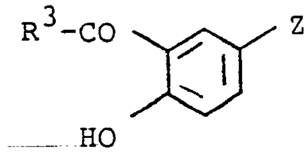

II

worin
Z -CHOH-CHR$^1$-X oder

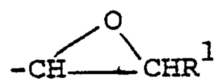

und
X Cl, Br, J oder eine reaktionsfähig funktionell abgewandelte Hydroxygruppe
bedeuten und
$R^1$ und $R^3$ die angegebenenen Bedeutungen haben,
mit einer Base der allgemeinen Formel III
H-$R^2$ III
worin
$R^2$ die angegebene Bedeutung hat
umsetzt
oder daß man eine Verbindung, die sonst der Formel I entspricht, aber an Stelle eines oder mehrerer H-Atome eine oder mehrere reduzierbare Gruppen und/oder C-C-Bindungen und/oder C-N-Bindungen enthält, reduziert und/oder daß man gegebenenfalls einen Ester der Formel I ($R^3$ = Alkoxy mit 1-4 C-Atomen) durch Reaktion mit einer Verbindung der Formel $R^4$-$NH_2$, worin $R^4$ H oder Alkyl mit 1-4 C-Atomen bedeutet, in ein Amid der Formel I ($R_3$ = $NH_2$ oder Alkylamino mit 1-4 C-Atomen) umwandelt und/oder eine Base der Formel I durch Behandeln mit einer Säure in eines ihrer physiologisch unbedenklichen Säureadditionssalze überführt.

In den Verbindungen der Formel II steht X vorzugsweise für Cl, Br, J oder Arylsulfonyloxy mit 6-10 C-Atomen wie Benzol- oder p-Toluolsulfonyloxy.

Die Verbindungen der Formel I können nach an sich bekannten Methoden dargestellt werden, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, wie sie für die nachfolgend beschriebenen Umsetzungen an sich bekannt und geeignet sind. Dabei kann man auch von an sich bekannten, hier nicht näher erwähnten Varianten Gebrauch machen.

Die Ausgangsstoffe können gewünschtenfalls auch in situ hergestellt werden, derart, daß man sie aus dem Reaktionsgemisch nicht isoliert, sondern sofort weiter zu den Verbindungen der Formel I umsetzt.

Eine Herstellung der Salicylsäurederivate der Formel I aus den Verbindungen der Formeln II und III erfolgt zweckmäßig in Gegenwart eines inerten Lösungsmittels, z. B. eines Alkohols wie Methanol, Ethanol oder Isopropanol oder eines Ethers wie Diethylether, Tetrahydrofuran oder Dioxan bei Temperaturen zwischen etwa 0 und 120 °, vorzugsweise zwischen 20 und 100 °. Es kann von Vorteil sein, eine Base wie Triethylamin oder Pyridin zuzusetzen. Man kann auch ohne Lösungsmittel arbeiten oder einen Überschuß der Base III als Lösungsmittel verwenden.

Die Ausgangsstoffe der Formeln II und III sind größtenteils bekannt. Sofern sie nicht bekannt sind, können sie leicht in Analogie zu an sich bekannten Methoden hergestellt werden. Beispielsweise kann man Salicylsäurederivate der Formel o-$R^3$-CO-$C_6H_4$-OH mit Chloracetylchlorid oder Bromacetylbromid/$AlCl_3$ zu den entsprechenden 5-Haloacetyl-salicylsäurederivaten acylieren und diese zu den 5-Haloethanolen der Formel II (Z = -CHOH-$CH_2$Cl bzw. -CHOH-$CH_2$Br) reduzieren. Entsprechend sind mit Halopropionylhalogeniden die 5-Halopropionylsalicylsäurederivate und die entsprechenden 5-Halopropanole zugänglich. Die

Epoxide der Formel II

(Z = 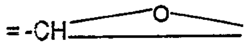) sind aus den Halo-alkoholen durch Dehydrohalogenierung erhältlich.

Bevorzugte Ausgangsstoffe zur Herstellung der Verbindungen der Formel I durch Reduktion entsprechen der Formel IV

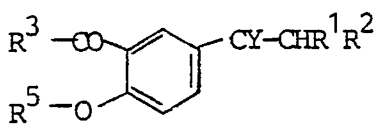

IV

worin

Y = O oder (H, OH) und

$R^5$ H oder eine hydrogenolytisch abspaltbare Gruppe, bevorzugt Benzyl,

bedeuten,

worin jedoch nicht gleichzeitig Y = (H, OH) und $R^5$ = H sind.

Die Reduktion kann z. B. erfolgen durch katalytische Hydrierung bei Temperaturen zwischen etwa 0 ° und etwa 200 ° sowie Drucken zwischen etwa 1 und 200 bar in einem inerten Lösungsmittel, z. B. einem der genannten Alkohole oder Ether, einem Ester wie Ethylacetat oder einer Carbonsäure wie Essigsäure. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können. Ketogruppen können auch mit komplexen Metallhydriden reduziert werden, wobei natürlich die anderen funktionellen Gruppen im Molekül nicht angegriffen werden dürfen. Besonders gut eignet sich $NaBH_4$ als Reduktionsmittel, das in einem der genannten Alkohole, falls erwünscht unter Zusatz weiterer Lösungsmittel wie Wasser und/oder Tetrahydrofuran bei Temperaturen zwischen etwa -10 und +50 ° zur Anwendung kommen kann. Weitere geeignete Reduktionsmittel sind z. B. $LiBH_4$, $Zn(BH_4)_2$, $LiAlH_4$, Triisobutylaluminiumhydrid in Ethern wie Tetrahydrofuran oder Dioxan. Besonders Salicylsäureester der Formel I ($R^3$ = Alkoxy mit 1-4 C-Atomen) lassen sich mit Vorteil auf diese Weise herstellen.

Die Ausgangsstoffe der Formel IV (Y = =O) sind z. B. erhältlich durch Reaktion von Salicylsäurederivaten der Formel o-$R^3$-CO-$C_6H_4$-OH mit Bromacetylbromid zu den entsprechenden 5-Bromacetyl-salicylsäurederivaten und Umsetzung mit III.

Ein Ester der Formel I ($R^3$ = Alkoxy mit 1-4 C-Atomen) kann mit einer Base der Formel $R^4$-$NH_2$ in das entsprechende Amid der Formel I ($R^3$ = $NH_2$ oder Alkyl mit 1-4 C-Atomen) umgewandelt werden, zweckmäßig in Gegenwart eines inerten Lösungsmittels, z. B. eines der angegebenen Alkohole, bei Temperaturen zwischen etwa -20 und +80 °.

Eine Base der Formel I kann mit einer Säure in das zugehörige Säureadditionssalz übergeführt werden. Für diese Umsetzung kommen Säuren in Frage, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Aepfelsäure, Benzoesäure, Salicylsäure, 2-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I können ein oder mehrere Asymmetriezentren besitzen. Sie können daher bei ihrer Herstellung als Racemate oder, falls optisch aktive Ausgangstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B.

optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger-oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Säureadditionssalze.

Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder.

Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Gegenstand der Erfindung ist ferner die Verwendung der Verbindungen der Formel I und ihrer physiologisch unbedenklichen Säureadditionssalze bei der therapeutischen Behandlung des menschlichen oder tierischen Körpers und bei der Bekämpfung von Krankheiten, insbesondere von allen Formen der Hypertonie und koronaren Herzerkrankungen, ferner von kardialen, nephrogenen oder hepatogenen Ödemen.

Dabei werden die erfindungsgemäßen Substanzen in der Regel in Analogie zu bekannten, im Handel befindlichen Präparaten ähnlicher Indikation (z. B. Molsidomin, Dihydralazin, Trichlormethiazid oder Hydrochlorothiazid) verabreicht, vorzugsweise in Dosierungen zwischen etwa 1 und 100 mg, insbesondere zwischen 5 und 50 mg pro Dosierungseinheit. Die tägliche Dosierung liegt vorzugsweise zwischen etwa 0,02 und 5 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jeder der in den folgenden Beispielen genannten Verbindungen der Formel I ist zur Herstellung von pharmazeutischen Zubereitungen besonders geeignet.

In den nachfolgenden Beispielen bedeutet "übliche Aufarbeitung":
Man gibt, falls erforderlich, Wasser, Natriumbicarbonat-oder Natriumcarbonatlösung oder verdünnte Natronlauge hinzu, extrahiert mit einem organischen Lösungsmittel wie Chloroform, trennt ab, dampft den organischen Extrakt ein und reinigt durch Chromatographie und/oder Kristallisation der Base oder eines ihrer Salze. Temperaturen sind in Celsiusgraden angegeben.

Beispiel 1

Man löst 18,9 g 1-(4-Methyl-2-thiazolyl)-piperazin und 26 g 5-(2-Brom-1-hydroxyethyl)-salicylamid (erhältlich durch Acylierung von Salicylamid mit Bromacetylbromid/$AlCl_3$ zu 5-Bromacetyl-salicylamid und nachfolgende Reaktion mit $NaBH_4$) in 400 ml Ether, fügt 15 ml Triethylamin hinzu, kocht 2 Std., arbeitet wie üblich auf und erhält 5-[1-Hydroxy-2-(4-(4-Methyl-2-thiazolyl)-piperazino)-ethyl]-salicylamid ("A"), F. 182° Dihydrochlorid, F. 244-245°.

Beispiele 2 bis 9

Analog Beispiel 1 erhält man mit 1-Tetrahydrofuroylpiperazin, 4-(4-Methyl-2-thiazolyl)-homopiperazin, 4-Benzamidopiperidin, 6,7-Dimethoxy-1,2,3,4-tetrahydroisochinolin, Imidazol, Tribromimidazol, 2-(3-Indolyl)-1,1-dimethyl-ethylamin bzw. aus 5-(3-Brom-1-hydroxypropyl)-salicylamid mit 1-(4-Methyl-2-thiazolyl)-piperazin:

2. 5-[1-Hydroxy-2-(4-tetrahydrofuroyl-piperazino)ethyl]-salicylamid.
3. 5-[1-Hydroxy-2-(4-(4-methyl-2-thiazolyl)-homopiperazino)-ethyl]-salicylamid, F. 195°.
4. 5-[1-Hydroxy-2-(4-benzamidopiperidino)-ethyl]salicylamid, F. 202°.
5. 5-[1-Hydroxy-2-(6,7-dimethoxy-1,2,3,4-tetrahydroisochinolino)-ethyl]-salicylamid, F. 187°.
6. 5-[1-Hydroxy-2-(1-imidazolyl)-ethyl]-salicylamid.
7. 5-[1-Hydroxy-2-(tribrom-1-imidazolyl)-ethyl]salicylamid, F.216 °.
8. 5-[1-Hydroxy-2-(2-(3-indolyl)-1,1-dimethyl-ethylamino)-ethyl]-salicylamid, Hydrochlorid, F. 248°.
9. 5-[1-Hydroxy-2-(4-(4-methyl-2-thiazolyl)-piperazino)-propyl]-salicylamid, Dihydrochlorid-monohydrat, F. 186°.

Beispiel 10

Eine Lösung von 18,9 g 4-(4-Methyl-2-thiazolyl)-piperazin und 17,9 g 5-Epoxyethyl-salicylamid [erhältlich durch Dehydrobromierung von 5-(2-Brom-1-hydroxyethyl)salicylamid] in 300 ml Tetrahydrofuran wird über Nacht stehengelassen. Nach üblicher Aufarbeitung erhält man "A", F. 182°.

Analog sind die in Beispiel 2 bis 9 angegebenen Verbindungen erhältlich.

Beispiel 11

Eine Lösung von 37,5 g 5-[4-(4-Methyl-2-thiazolyl)-piperazino-acetyl]-salicylsäuremethylester [F.120 °, erhältlich durch 2 std. Kochen von 4-(4-Methyl-2-thiazolyl)-piperazin mit 5-Bromacetyl-salicylsäuremethylester in Ether in Gegenwart von Triethylamin] in 100 ml Ethanol, 50 ml Wasser und 300 ml Tetrahydrofuran wird unter Argon bei -5 bis 0 ° portionsweise mit $NaBH_4$ bis zur vollendeten Umsetzung (DC-Kontrolle) versetzt. Man rührt noch 30 Min. bei 20 °, säuert mit Essigsäure an, engt ein, arbeitet wie üblich auf und erhält 5-[1-Hydroxy-2-(4-(4-methyl-2-thiazolyl)-piperazino)-ethyl]-salicylsäuremethylester, F. 121°. Dihydrochlorid, F. 186°.

Beispiele 12 bis 19

Analog Beispiel 11 erhält man aus
5-(4-Tetrahydrofuroyl-piperazino-acetyl)-salicylsäuremethylester
5-[4-(4-Methyl-2-thiazolyl)-homopiperazino-acetyl]-salicylsäuremethylester
5-(4-Benzamidopiperidino-acetyl)-salicylsäuremethylester (F. 158°)
5-(6,7-Dimethoxy-1,2,3,4-tetrahydroisochinolino-acetyl)-salicylsäuremethylester
5-(1-imidazolyl-acetyl)-salicylsäuremethylester (F.142°) 5-(Tribrom-1-imidazolyl-acetyl)-salicylsäuremethylester (F. 174°)
5-[2-(3-Indolyl)-1,1-dimethyl-ethylamino-acetyl]-salicylsäuremethylester
[2-(4-(4-Methyl-2-thiazolyl)-piperazino)-propionyl]-salicylsäuremethylester (Dihydrochlorid, F.191°)
mit $NaBH_4$:

12. 5-[1-Hydroxy-2-(4-tetrahydrofuroyl-piperazino)-ethyl]-salicylsäuremethylester, Hydrochlorid, F.218°.
13. 5-[1-Hydroxy-2-(4-(4-methyl-2-thiazolyl)-homopiperazino)-ethyl]-salicylsäuremethylester, Dihydrochlorid-monohydrat, F. 154°.
14. 5-[1-Hydroxy-2-(4-benzamidopiperidino)-ethyl]-salicylsäuremethylester, Hydrochlorid, F. 246°.
15. 5-[1-Hydroxy-2-(6,7-dimethoxy-1,2,3,4-tetrahydroisochinolino)-ethyl]-salicylsäuremethylester.
16. 5-[1-Hydroxy-2-(1-imidazolyl)-ethyl]-salicylsäuremethylester.
17. 5-[1-Hydroxy-2-(tribrom-1-imidazolyl)-ethyl]-salicylsäuremethylester, F. 175°.

18. 5-[1-Hydroxy-2-(2-(3-indolyl)-1,1-dimethyl-ethylamino)-ethyl]-salicylsäuremethylester, Hydrochlorid, F. 201°.

19. 5-[1-Hydroxy-2-(4-(4-methyl-2-thiazolyl)-piperazino)-propyl]-salicylsäuremethylester, Dihydrochlorid, F. 186°.

Beispiele 20 bis 25

Analog Beispiel 11 erhält man aus den entsprechenden Ketoestern mit $NaBH_4$:

20. 5-[1-Hydroxy-2-(4-(4-methyl-2-thiazolyl)-piperazino)-ethyl]-salicylsäure-ethylester.

21. 5-[1-Hydroxy-2-(4-(4-methyl-2-thiazolyl)-piperazino)-ethyl]-salicylsäure-propylester, 22. 5-[1-Hydroxy-2-(4-(4-methyl-2-thiazolyl)-piperazino)-ethyl]-salicylsäure-isopropylester.

23. 5-[1-Hydroxy-2-(4-(4-methyl-2-thiazolyl)-piperazino)-ethyl]-salicylsäure-butylester.

24. 5-[1-Hydroxy-2-(4-(4-methyl-2-thiazolyl)-piperazino)-ethyl]-salicylsäure-isobutylester.

25. 5-[1-Hydroxy-2-(4-(4-methyl-2-thiazolyl)-piperazino)-ethyl]-salicylsäure-sek.-butylester.

Beispiel 26

Man hydriert eine Lösung von 10 g 2-Benzyloxy-5-[1-hydroxy-2-(4-(4-methyl-2-thiazolyl)-piperazino)-ethyl]-benzamid in 200 ml Ethanol an $PtO_2$ bei 20 ° und 2 bar bis zur Aufnahme der berechneten Menge Wasserstoff, arbeitet wie üblich auf und erhält "A", F. 182°.

Beispiel 27

Man hydriert eine Lösung von 10 g 5-[4-(4-Methyl-2-thiazolyl)-piperazino-acetyl]-salicylamid [erhältlich aus 4-(4-Methyl-2-thiazolyl)-piperazin und 5-Bromacetyl-salicylamid] in 200 ml Methanol an 1 g 10 %iger Pd-Kohle bei 40 ° und 1 bar bis zur Aufnahme der berechneten Menge Wasserstoff, filtriert, dampft ein und erhält "A", F. 182°.

Beispiel 28

Eine Lösung von 10 g 5-[1-Hydroxy-2-(4-(4-Methyl-2-thiazolyl)-piperazino)-ethyl]-salicylsäuremethylester und 10 g $NH_4Cl$ in einem Gemisch aus 100 ml gesättigter methanolischer $NH_3$-Lösung und 100 ml 25 %igen wässeriger $NH_3$-Lösung wird 48 Stunden bei 20 ° stehengelassen, eingeengt und wie üblich aufgearbeitet. Man erhält "A", F. 182°. Dihydrochlorid, F. 244-245°.

Analog erhält man aus den in den Beispielen 12 bis 19 angegebenen Methylestern mit $NH_3$ die in den Beispielen 2 bis 9 angegebenen Amide.

Beispiel 29

Eine Lösung von 200 mg Na in 120 ml Methanol wird mit 10 g 5-[1-Hydroxy-2-(4-(4-methyl-2-thiazolyl)-piperazino)-ethyl]-salicylsäuremethylester versetzt. Man leitet 30 Min. lang Methylamin ein, wobei man die Innentemperatur unterhalb 30 ° hält. Nach 12 std. Stehen engt man ein, arbeitet wie üblich auf und erhält 5-[1-Hydroxy-2-(4-(4-methyl-2-thiazolyl)-piperazino)-ethyl]-salicylsäure-N-methylamid, F. 252°.

Beispiele 30 bis 37

Analog Beispiel 29 erhält man aus den entsprechenden Methylestern mit Methylamin:

30. 5-[1-Hydroxy-2-(4-tetrahydrofuroyl-piperazino)-ethyl]-salicylsäure-N-methylamid.

31. 5-[1-Hydroxy-2-(4-(4-methyl-2-thiazolyl)-homopiperazino)-ethyl]-salicylsäure-N-methylamid.

32. 5-[1-Hydroxy-2-(4-benzamidopiperidino)-ethyl]salicylsäure N-methylamid.

33. 5-[1-Hydroxy-2-(6,7-dimethoxy-1,2,3,4-tetrahydroisochinolino)-ethyl]-salicylsäure-N-methylamid.
34. 5-[1-Hydroxy-2-(1-imidazolyl)-ethyl-salicylsäure-N-methylamid.
35. 5-[1-Hydroxy-2-(tribrom-1-imidazolyl)-ethyl]salicylsäure-N-methylamid.
36. 5-[1-Hydroxy-2-(2-(3-indolyl)-1,1-dimethyl-ethylamino)-ethyl]-salicylsäure-N-methylamid.
37. 5-[1-Hydroxy-2-(4-(4-methyl-2-thiazolyl)-piperazino-propyl]-salicylsäure-N-methylamid.

Beispiele 38 bis 41

Analog Beispiel 29 erhält man aus 5-[1-Hydroxy-2-(4-(4-methyl-2-thiazolyl)-piperazino)-ethyl]-salicylsäuremethylester mit den entsprechenden Alkylaminen:

38. 5-[1-Hydroxy-2-(4-(4-methyl-2-thiazolyl)-piperazino)-ethyl]-salicylsäure-N-ethylamid.
39. 5-[1-Hydroxy 2-(4 (4-methyl-2 thiazolyl)-piperazino)-ethyl]-salicylsäure-N-propylamid.
40. 5-[1-Hydroxy-2-(4-(4-methyl-2-thiazolyl)-piperazino)-ethyl]-salicylsäure-N-isopropylamid.
41. 5-[1-Hydroxy-2-(4-(4-methyl-2-thiazolyl)-piperazino)-ethyl]-salicylsäure-N-butylamid.

Die nachstehenden Beispiele betreffen pharmazeutische Zubereitungen, die Verbindungen der Formel I enthalten:

Beispiel A: Tabletten

Ein Gemisch von 1 kg "A"-Dihydrochlorid, 4 kg Lactose, 1,2 kg Maisstärke, 0,2 kg Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Weizenstärke, Talk, Tragant und Farbstoff überzogen werden.

Beispiel C: Kapseln

5 kg "A"-Dihydrochlorid werden in üblicher Weise in Hartgelatinekapseln gefüllt, so daß jede Kapsel 20 mg des Wirkstoffs enthält.

Beispiel D: Ampullen

Eine Lösung von 1 kg "A"-Dihydrochlorid in 30 l zweifach destilliertem Wasser wird steril filtriert, in Ampullen abgefüllt, unter sterilen Bedingungen lyophilisiert und steril verschlossen. Jede Ampulle enthält 5 mg Wirkstoff.

Analog sind Tabletten, Dragees, Kapseln oder Ampullen erhältlich, die einen oder mehrere der übrigen Wirkstoffe der Formel I und/oder ihre physiologisch unbedenklichen Säureadditionssalze enthalten.

## Ansprüche

1. Salicylsäurederivate der allgemeinen Formel I

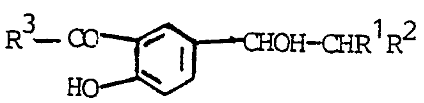

I

worin
$R^1$ H oder $CH_3$,
$R^2$ 4-(4-Methyl-2-thiazolyl)-piperazino, 4-(Tetrahydro-2-furoyl)-piperazino, 4-(4-Methyl-2-thiazolyl)-homopiperazino, 4-Benzamidopiperidino, 6,7-Dimethoxy-1,2,3,4-tetrahydroisochinolino, 1-Imidazolyl, Tribrom-1-imidazolyl oder 2-(3-Indolyl)-1,1-dimethyl-ethylamino und
$R^3$ Alkoxy mit 1-4 C-Atomen, $NH_2$ oder Alkylamino mit 1-4 C-Atomen
bedeuten,
sowie deren physiologisch unbedenkliche Säureadditionssalze.

2. 5-[1-Hydroxy-2-(4-(4-methyl-2-thiazolyl)-piperazino)-ethyl]-salicylamid und dessen physiologisch unbedenkliche Säureadditionssalze.

3. Verfahren zur Herstellung der Salicylsäurederivate der allgemeinen Formel I nach Anspruch 1 sowie ihrer physiologisch unbedenklichen Säureadditionssalze, dadurch gekennzeichnet, daß man ein Salicylsäurederivat der allgemeinen Formel II

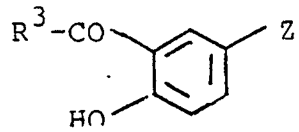

II

worin
Z -CHOH-CHR$^1$-X oder

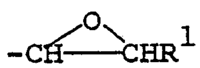

und
X Cl, Br, J oder eine reaktionsfähig funktionell abgewandelte Hydroxygruppe
bedeuten und
$R^1$ und $R^3$ die angegebenen Bedeutungen haben,
mit einer Base der allgemeinen Formel III
H-$R^2$ III
worin
$R^2$ die angegebene Bedeutung hat
umsetzt
oder daß man eine Verbindung, die sonst der Formel I entspricht, aber an Stelle eines oder mehrerer H-Atome eine oder mehrere reduzierbare Gruppen und/oder C-C-Bindungen und/oder C-N-Bindungen enthält, reduziert
und/oder daß man gegebenenfalls einen Ester der Formel I ($R^3$ = Alkoxy mit 1-4 C-Atomen) durch Reaktion mit einer Verbindung der Formel $R^4$-$NH_2$, worin $R^4$ H oder Alkyl mit 1-4 C-Atomen bedeutet, in ein Amid der Formel I ($R^3$ = $NH_2$ oder Alkylamino mit 1-4 C-Atomen) umwandelt und/oder eine Base der Formel I durch Behandeln mit einer Säure in eines ihrer physiologisch unbedenklichen Säureadditionssalze überführt.

4. Verfahren zur Herstellung pharmazeutischer Zubereitungen, dadurch gekennzeichnet, daß man eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I nach Patentanspruch 1 zur Bekämpfung von Krankheiten.

7. Verwendung von Verbindungen der Formel I bei der Senkung des Blutdrucks.